# EUROPÄISCHE PATENTSCHRIFT

(11) **EP 0 647 850 B1**
(45) Veröffentlichungstag und Bekanntmachung des Hinweises auf die Patenterteilung: **17.01.2001**
(21) Anmeldenummer: 94890160.8
(22) Anmeldetag: 05.10.1994
(51) Int. Cl.: G01N 33/92, G01N 33/53

(54) **Aufbereitung von lipoproteinhältigen Proben**
Treatment of lipoprotein containing samples
Traitement d'échantillons contenants les lipoprotéines

(30) Priorität: 11.10.1993 AT 204393
(43) Veröffentlichungstag der Anmeldung: 12.04.1995
(73) Patentinhaber: Technoclone Gesellschaft m.b.H., 1235 Wien (AT)
(72) Erfinder: Molinari, Ewald, Dr., A-2340 Mödling (AT); Pichler, Peter, Dipl.-Ing., A-1080 Wien (AT); Lang, Hartmut, Dr., A-2560 Grillenberg (AT)
(74) Vertreter: Patentanwälte BARGER, PISO & PARTNER

(56) Entgegenhaltungen:
- EP-A- 0 058 959
- DE-A- 3 329 952
- DE-A- 3 330 770
- CLINICA CHIMICA ACTA, Bd. 135, 1983, AMSTERDAM, Seiten 203-208, XP000195977 CAZZOLATO: "The determination of lipoprotein Lp(a) by rate and endpoint nephelometry"

## Beschreibung

Die Erfindung betrifft ein Probenaufbereitungsmittel für lipoproteinhältige Proben und zur immunchemischen Bestimmung eines Antigens sowie ein Set zur Herstellung eines Probenaufbereitungsmittels. Ebenso betrifft die Erfindung ein Verfahren zur immunchemischen Bestimmung eines Antigens sowie ein Verfahren zur Verringerung oder Beseitigung von Trübungen in einer biologischen Flüssigkeit, z.B. in einer Blut- oder Plasmaprobe.

Ein Lipoprotein ist ein Partikel mit einem Kern aus hydrophoben Lipiden (Cholesterin, Cholesterinester, Phosphatide, Triglyceride), der von einer Hülle aus polaren Lipiden und Apoproteinen A-1, A-2, A-4, B-48, B-100, C und E umgeben ist. Die Lipoproteine solubilisieren einerseits stark hydrophobe Lipide, und enthalten andererseits Signale, die den Transport bestimmter Lipide an spezifischen Zellen und Geweben regulieren.

Lipoproteine können in verschiedene Dichteklassen aufgeteilt werden:

Chylomikronen, VLDL ("very low density lipoproteins"), IDL ("intermediate density lipoproteins"), LDL ("low density lipoproteins") und HDL ("high density lipoproteins")

Abhängig vom Proteinanteil zeigen die Lipoproteine in der Elektrophorese eine unterschiedliche Mobilität und werden in Beta-Lipoproteine, Prä-Beta-Lipoproteine und Alpha-Lipoproteine eingeteilt. LP-X ist ein pathologisches Lipoprotein, das zu einem hohen Anteil aus Phosphatiden und Cholesterinester besteht.

Lp(a) ist ein weiteres Lipoprotein, das in das Arteriosklerosegeschehen involviert ist. Die Struktur des Lp(a) läßt sich vom Aufbau des LDL Partikels ableiten. Lp(a) ein LDL-Partikel, das über Disulfidbrücken mit dem hoch glykosilierten Apo-Lipoprotein (a) verbunden ist. Lp(a) weist sowohl inter- als auch intraindividuelle Heterogenität in seiner Lipid- und Proteinzusammensetzung auf. Es stellt einen genetisch determinierten, unabhängigen Risikofaktor für Arteriosklerose dar.

Die Bestimmung von Lipoproteinen in einer biologischen Flüssigkeit, wie einer Blut- oder Plasmaprobe gewinnt im Rahmen der Vorsorgeuntersuchungen zur Erfassung des Risikos von Herz- und Kreislauferkrankungen, aber auch im Rahmen von Therapien zur Kontrolle des Lipoproteingehalts zunehmend an Bedeutung. Lipoproteine werden im allgemeinen durch immunchemische Bestimmung erfaßt. Dafür werden Antikörper gegen das zu bestimmende Lipoprotein eingesetzt und die entstandenen Immunkomplexe bestimmt. Die Detektion der entstandenen Immunkomplexe erfolgt dabei durch optische Methoden, beispielsweise durch Erfassung der Zunahme der Trübung in einer Probe. Voraussetzung für die dafür notwendigen optischen Tests ist eine möglichst klare Probenlösung, um die Empfindlichkeit der Bestimmung zu maximieren.

Die Eigentrübung von Seren stellt vor allem bei lipämischen oder hyperlipoproteinämischen Seren ein Problem dar. Auch bei geringem Lipämiegrad kann nämlich durch die Trübung die Detekion von geringen Mengen an Lipoproteinen unmöglich werden.

So ist beispielsweise die Detekion von Immunpräzipitaten, die nephelometrisch oder turbidimetrisch gemessen werden, durch die Eigentrübungen des Probenmaterials empfindlich gestört.

Insbesondere stößt die turbidimetrische Bestimmung von Lp(a) wegen der Größe des Partikels bzw. seiner strukturellen Heterogenität, besonders bei Proben mit hoher Eigentrübung (Chylomikronen, Triglyceride) und einmal eingefrorenen und wieder aufgetauten Proben immer wieder auf Schwierigkeiten. Bei solchen Proben werden oft falsch hohe Lp(a) Konzentrationen gefunden. Gleichgelagerte Schwierigkeiten ergeben sich bei der nephelometrischen Bestimmung des Lp(a).

Die Beseitigung von Trübungen in einem Serum ist daher für die klinische Analyse, insbesondere für die Bestimmung von Lipoproteinen von außerordentlicher Bedeutung.

Ein gängiges Maß für die Trübung, die eine Lösung aufweist ist das LSU (light scattering Unit). Im allgemeinen gilt eine Lösung als trüb, wenn sie bei der Messung im Nephelometer bei 340 nm und einer Schichtdicke von 1 cm ein Meßsignal von mehr als 70 LSU aufweist. Eine Messung der Antigen-Antikörper-Reaktion wird bei 70 LSU und darüber deutlich verfälscht. Zur klinischen Analyse sollten Proben eine maximale Trübung von vorzugsweise zwischen 30 und 40 LSU aufweisen.

Zur Beseitigung der störenden Trübungen in lipämischen Proben wird beispielsweise in der Clinica Chimica Acta (135 (1983) 203 - 208) eine Behandlung mit Lipasen oder eine Hochgeschwindigkeitszentrifugation empfohlen. Von der Verwendung von Detergentien wird aber unbedingt abgeraten, da diese zwar die Eigentrübung der Probe herabsetzen, aber auch zu einer starken Reduktion der immunchemischen Antwort führen. Als Reaktionsbeschleuniger für die immunchemische Reaktion wird Polyethylenglycol vorgeschlagen.

Bei der Lyophilisation von Kontrollseren kommt es insbesondere bei lipämischen Proben durch den Vorgang des Lyophilisieren und des anschließenden Rekonstituierens der ursprünglich klaren Seren zur Entstehung einer Trübung. Als Vorkehrung zur Verhinderung der Trübung wird im EP 0 141 922 die Zugabe von einem Detergens und Prolin vor der Lyophilisierung des Kontrollserums vorgeschlagen. Die Seren werden mit Prolin und Natriumdesoxycholat versetzt und lyophilisiert. Nach Rekonstitution des Lyophilisates konnte festgestellt werden, daß die klaren Seren nicht trüb geworden waren. Da bei diesem Haltbarmachen von Kontrollseren immer von klaren Seren ausgegangen wird, konnte eine Klärung der Lösung, d.h. eine Verringerung der Trübung durch Detergens/Prolin nicht erfolgen.

Auch in der EP 0 058 959 sind diverse Zusätze zur Herstellung von Kontrollseren genannt, die nach Lyophilisierung und Rekonstitution klar bleiben sollen. Als Mittel zur Verhinderung einer Trübung bedingt durch die Änderung des Aggregatzustandes werden etwa organische, nicht zuckerartige Substanzen wie Methanol, Alanin, Triethylenglycol, Valin, Acetat, Lactat oder Natrium-2-hydroxymethylbutyrat vorgeschlagen. Aber auch in der EP 0 058 959 wurde keine Möglichkeit geoffenbart, eine zu untersuchende Probe zu behandeln, um eine ungestörte Bestimmung eines Lipoproteingehalts durchführen zu können.

Die Erfindung stellt sich zur Aufgabe, eine Technologie zur Verfügung zu stellen, mit der einerseits eine eventuell vorhandene Eigentrübung in biologischen Flüssigkeiten verringert oder beseitigt und andererseits die möglichst unverfälschte Bestimmung eines Antigens in einer Probe ermöglicht wird.

Die Aufgabe wird erfindungsgemäß durch ein Mittel gelöst, welches zur Probenaufbereitung eine Aminosäure enthält, die eine Trübung in der Probe vermindert oder beseitigt und gleichzeitig die immunchemische Bestimmung eines Antigens vorzugweise eines Lipoproteins mit Hilfe eines im Mittel enthaltenen Antikörpers gegen das zu bestimmende Antigen unbeeinflußt läßt. Das erfindungsgemäße Mittel weist vorteilhafterweise einen Gehalt eines Reaktionsbeschleunigers auf, beispielsweise Polyethylenglykol. Die Aminosäure liegt in einem gepufferten Medium, vorzugsweise in einer Konzentration von 0,05 - 3 M, am meisten bevorzugt 0,1 - 0,5 M vor. Es hat sich herausgestellt, daß vor allem Prolin zur Beseitigung der Trübung in einer lipämischen bzw. trüben Probe hervorragend geeignet ist, unabhängig von der Vorbehandlung der Probe.

Eine bevorzugte Ausführungsform des Mittels enthält Antikörper gegen ein Lipoprotein und wird zur immunchemischen Bestimmung dieses Lipoproteins verwendet.

Beispielsweise kann das erfindungsgemäße Mittel zur Bestimmung von Lp(a) eingesetzt werden, wobei das Mittel in diesem Fall Lp(a) Antikörper, vorzugsweise ein Lp(a)-Antiserum enthält. Das erfindungsgemäße Mittel kann durch Modifikation von käuflich erhältlichen Reagenzien bzw. Reagenskomponenten in einfacher Weise hergestellt werden.

Das erfindungsgemäße Mittel ermöglicht die ungestörte Bestimmung vorzugsweise von Lipoproteinen in einer Probe, gleichgültig, ob die Probe zuvor tiefgefroren, gekühlt gelagert oder frisch abgenommen wurde. Als Probe kann insbesondere eine biologische Flüssigkeit herangezogen werden, beispielsweise eine Blut-, Serum- oder Plasmaprobe, die durch einen Lipoproteingehalt optisch trüb ist.

Es ist überraschend, daß das erfindungsgemäße Mittel die Empfindlichkeit der Bestimmung von beispielsweise Lp(a) wesentlich erhöht, wobei gleichzeitig mit einer Referenzmethode gezeigt werden kann, daß die erhaltenen Ergebnisse von hoher Relevanz sind. Der Zusatz von Prolin zu einem Probenaufbereitungsmittel senkt deutlich den Blindwert einer Serumprobe und erhöht die nephelometrisch bestimmte Extinktionsdifferenz ab dem Zeitpunkt der Antikörperzugabe.

Der vorteilhafte Effekt von Aminosäuren, insbesondere Prolin, im erfindungsgemäßen Mittel ist umso überraschender, da Aminosäuren im allgemeinen im Zusammenhang mit Detergenzien nur zur Verhinderung einer Trübungsbildung in einem lyophilisierten Kontrollserum vorgeschlagen werden, jedoch derartige Zusätze (Detergenzien) zu einer Probe für eine immunchemische Bestimmung als störend betrachtet wurden. Außerdem werden solche Proben nicht lyophilisiert, wodurch keine Veranlassung bestand, eine Trübungsbildung während einer Lyophilisierung und Rekonstitution durch derartige Zusätze zu verhindern.

Auch beim erfindungsgemäßen Mittel ist es vorteilhaft, daß der Probe kein Tensid zugesetzt wird, um eine ungestörte immunchemische Bestimmung eines Antigens zu ermöglichen. Das erfindungsgemäße Mittel oder das Set zur Herstellung des Mittels sowie die Komponenten in diesem Set sind also vorteilhafterweise im wesentlichen frei von Tensiden. Als "im wesentlichen" tensidfrei werden Lösungen angesehen, die einen Tensidgehalt von weniger als etwa 0,01% aufweisen.

Eine immunchemische Bestimmung in einer Lösung mit einem Tensidgehalt von mehr als 0,05 % wird schon so deutlich durch das Tensid verfälscht, daß die erhaltenen Meßergebnisse nicht mehr aussagekräftig sind.

Es hat sich auch gezeigt, daß es vorteilhaft ist, das erfindungsgemäße Mittel als Set von zumindest zwei Komponenten zur Verfügung zu stellen. In einer ersten Komponente kann somit die Aminosäure und gegebenenfalls ein Reaktionsbeschleuniger in Gegenwart von Puffersubstanzen vorgelegt werden, eine zweite Komponente enthält Antikörper gegen das zu bestimmende Antigen.

Gemäß einem weiteren Aspekt betrifft die Erfindung ein Verfahren zur immunchemischen Bestimmung eines Antigens bei welchem einer zur immunchemischen Bestimmung vorgeschlagenen Probe eine Aminosäure zugesetzt wird, anschließend oder gleichzeitig Antikörper gegen das zu bestimmende Antigen in die Probe eingebracht werden und der gebildete Antikörper-Antigen-Komplex bestimmt wird.

Ein anderer Aspekt der Erfindung betrifft ein Verfahren zur Verringerung oder Beseitigung von Trübungen in einer trüben, biologischen Flüssigkeit welches dadurch gekennzeichnet ist, daß der trüben biologischen Flüssigkeit eine Aminosäure zugegeben wird. Dadurch wird gleichzeitig ein Verfahren zur Aufbereitung einer Probenflüssigkeit zur Verfügung gestellt.

Bei den erfindungsgemäßen Verfahren wird bevorzugt Prolin als Aminosäure verwendet.

Die erfindungsgemäßen Verfahren werden vorzugsweise bei Blut-bzw. Plasmaproben angewendet sowie bei zur immunchemischen Bestimmung vorgesehenen trüben, lipoproteinhältigen Proben eines Antigens.

Ein weiterer Aspekt der Erfindung betrifft die Verwendung einer Aminosäure, insbesondere Prolin, zur Verringerung oder Beseitigung von Trübungen in einer biologischen Flüssigkeit, vorzugsweise in einer zur immunchemischen Bestimmung vorgesehenen trüben, lipoproteinhältigen Probe eines Antigens, sowie die Verwendung einer Aminosäure, insbesondere Prolin, zur Aufbereitung von lipoproteinhältigen Proben, vorzugsweise in einer zur immunchemischen Bestimmung eines Antigens vorgesehenen Probe.

Die Erfindung wird durch die nachfolgenden Beispiele noch näher erläutert. Die Beispiele beschreiben die Bestimmung von Lp(a) in Serumproben mit Hilfe von entsprechend modifizierten, käuflich erhältlichen Einzelreagenzien von Immuno AG entsprechend einer gültigen Arbeitsvorschrift (Anti-Human Lp(a) v. Schaf, Reference Standard Lp(a) Human, Norm control Lp(a) Human). Die Extinktionsdifferenz wird mit Hilfe eines Photometers oder Analysenautomates erfaßt, in den angeführten Beispielen mit einem COBAMSIRA-Gerät (Hoffmann La Roche).

### Beispiel 1: Extinktionsverlauf der Eichkurve.

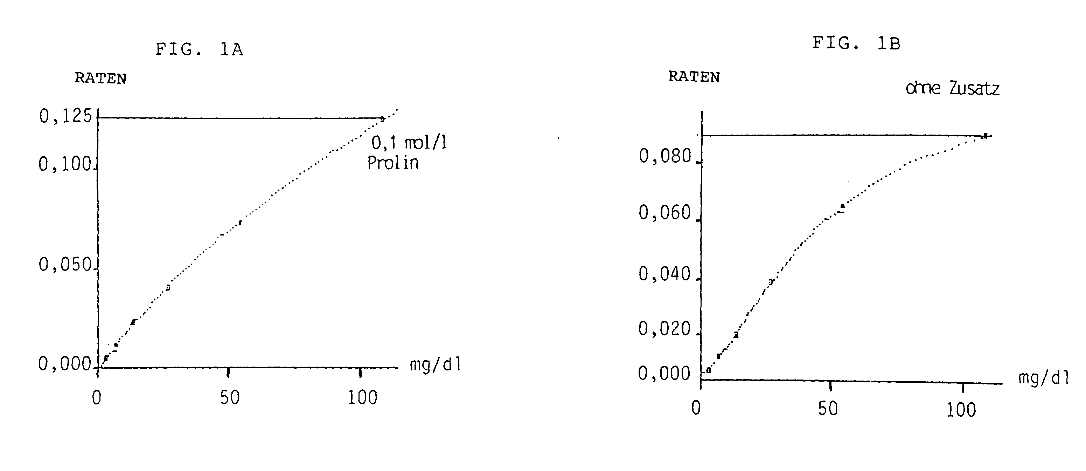

Von einer Reihe von Lp(a) Lösungen mit unterschiedlichen Konzentrationen an Lp(a) wurde die Extinktion gemessen und eine Eichkurve erstellt. Einer Probe enthaltend Lp(a) wird Verdünnungspuffer enthaltend 1 x PBS (1 x PBS : 10 mM Na/K-Phosphat, 0,8 % NaCl, 0,2 % HCl, pH 7,2) und 4 % PEG mit oder ohne Zusatz von 0,1 mol/l Prolin und nach 5 min eine Lp(a)-Antikörperlösung zugegeben. In Fig.1A ist der Extinktionsverlauf der Eichkurve mit dem Zusatz von 0,1 mol/l Prolin im Verdünnungsmedium und in Fig. 1B derselbe Verlauf ohne Zusatz wiedergegeben.

Wie ersichtlich weist der Calibrator mit der höchsten Lp(a) Konzentration, nämlich 108 mg/dl, eine Extinktion von 0,09 RATES gegenüber 0,125 RATES desselben Calibrators im Verdünnungsmedium mit 0,1 mol/l Prolin auf. Durch den Zusatz von Prolin wird also das Reaktionssignal verstärkt. RATES entsprechen dabei dem Meßsignal der Antigen-Antikörper-Reaktion nach Subtraktion des Meßsignals, das nach Zugabe des Verdünnungspuffers gegenüber der ursprünglichen Lösung aufgetreten ist.

Weiters wird aus dem Verlauf der Eichkurve ersichtlich, daß die Eichkurve ohne Zusatz im Verdünnungsmedium zu höheren Konzentrationen hin abflacht (Fig. 1B), während die in Fig. 1A gezeigte Kurve einen linearen Verlauf zeigt.

### Beispiel 2: Lp(a)-Bestimmung mit Vorinkubation

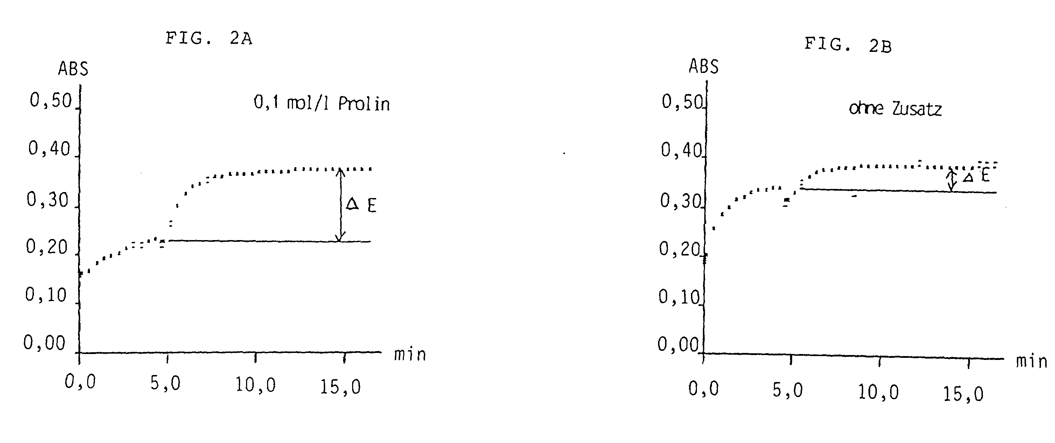

Eine Serumprobe mit hoher Eigentrübung wird mit Verdünnungspuffer enthaltend 0,1 mol/l Prolin 5 min inkubiert. Dann folgt eine Zugabe von Lp(a)-Antikörpern. Die Extinktion wird bei einer Wellenlänge von 340 nm gemessen und gegen die Zeiteinheit aufgetragen (Fig. 2A). Derselbe Versuch wurde auch ohne (Fig. 2B) Zugabe von Prolin im Verdünnungspuffer durchgeführt. Daraus ist ersichtlich, daß die Extinktion entsprechend dem Blindwert der Serumprobe von 0,36 (ohne Zusatz) auf 0,24 (mit Prolin) gesenkt werden konnte.

Die Extinktionsdifferenz ab dem Zeitpunkt der Antikörperzugabe ist im Reaktionsverlauf des Ansatzes mit Prolin (Fig.2A) um 200 % größer als im Ansatz ohne Prolin (Fig. 2B). Daraus errechnet sich eine Lp(a)-Konzentration von 103,3 mg/dl im Ansatz mit Prolin, jedoch gleichzeitig ein falsch niedriger Wert von 19,2 mg/dl im Ansatz ohne Prolin. Im allgemeinen wird eine Lp(a)-Konzentration von unter 30 als normal angesehen, darüber liegende Werte gelten als pathologisch. Im vorliegenden Fall wäre also die Probe aufgrund der konventionellen Meßmethoden als "normal" (19,2 mg/dl) angesehen worden, obgleich die richtige Konzentration (ermittelt mit Referenzmethode) einen deutlich pathologischen Wert darstellt.

### Beispiel 3: Lp(a)-Bestimmung mit Vorinkubation

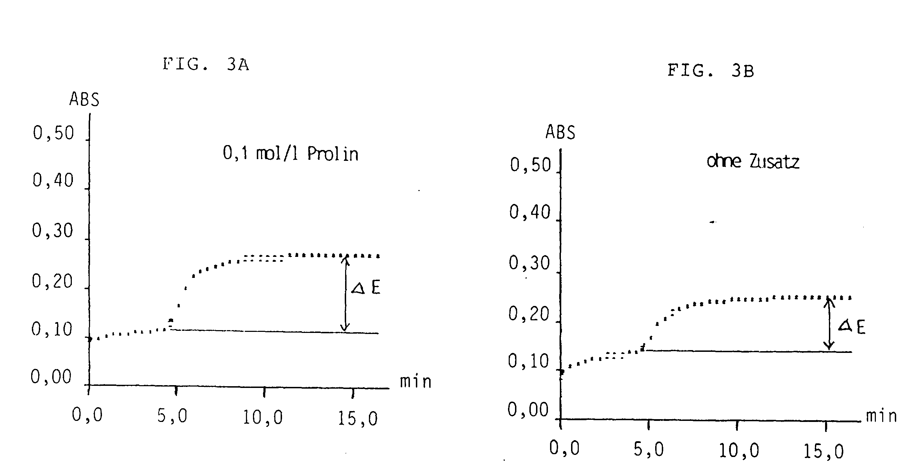

Analog zu Beispiel 2 wurde der Lp(a)-Gehalt einer Probe durch Zugabe von Lp(a)-Antikörpern turbidimetrisch bestimmt. Derselbe Reaktionsverlauf wie in Fig. 2A und 2B ist in den Fig. 3A und 3B mit dem Calibrator der höchsten Lp(a)-Konzentration (= 108 mg/dl) wiedergegeben. In diesem Beispiel konnte das Reaktionssignal (ohne Zusatz, Beispiel 3B) durch den Zusatz von 0,1 mol/1 Prolin um 50 % verstärkt werden.

### Beispiel 4: Korrelation zwischen frischen und aufgetauten Proben

Wie erwähnt ergaben sich bei einmal eingefrorenen und wieder aufgetauten Seren bei der turbidimetrischen Bestimmung des Lp(a) gegenüber frisch getesteten Proben wegen der Trübung des aufgetauten Probenmaterials Diskrepanzen.

Die erzielte Verbesserung durch Zusatz von Prolin zum Mittel soll durch das folgende Beispiel dokumentiert werden:

Ein Panel von 25 Serumproben wurde aliquotiert, der eine Teil noch am selben Tag der Blutabnahme getestet, der andere Teil eingefroren. Dieses Aliquot wurde am nächsten Tag aufgetaut und dann bestimmt.

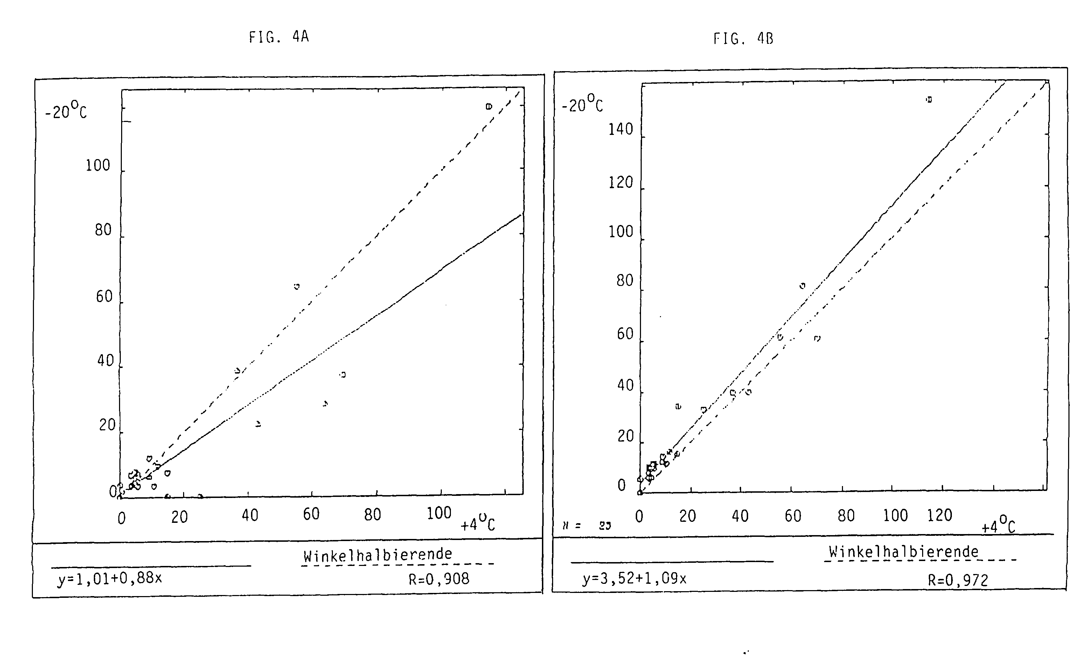

Die Ergebnisse der Bestimmungen ohne Prolin sind in Fig. 4A dargestellt. Die Korrelation zwischen den frisch getesteten und den aufgetauten Proben betrug R = 0,908 und die Geradengleichung lautet y = 1,01 + 0,88x. In einem Parallelansatz wurden die aufgetauten Proben mit Prolin bestimmt. Der Vergleich zu den frisch getesteten Proben ist in Fig. 4B dargestellt. Die Korrelation verbesserte sich auf R = 0,972, auch die Geradengleichung zeigt mit y = 3,52 + 1,09x eine deutliche Verbesserung der Übereinstimmung mit den Idealwerten mit der Steigung 1.

### Beispiel 5: Lp(a)-Bestimmung mittels Elektroimmundiffusion

Als Referenzmethode der immunologischen Lp(a)-Bestimmung dient die Elektroimmundiffusion.

Die in Beispiel 4 verwendeten eingefroreren und aufgetauten Proben wurden in einem Parallelansatz auch in der Elektroimmundiffusion getestet. (Methode nach Laurell, Anal. Biochem. 15 (1966), 45)

In Fig. 5A sind die Ergebnisse der turbidimetrischen Bestimmung ohne Prolin denen der Bestimmung durch Elektroimmundiffusion gegenübergestellt. Die Korrelation beträgt R = 0,918 und die Geradengleichung für die Elektroimmundiffusionskurve lautet y = 1,95 + 0,54x. (y = 1,01 + 0,88x für die turbidimetrische Bestimmung: Fig. 4A). Es besteht somit zwischen den beiden Methoden ein deutlicher Unterschied in den erhaltenen Ergebnissen.

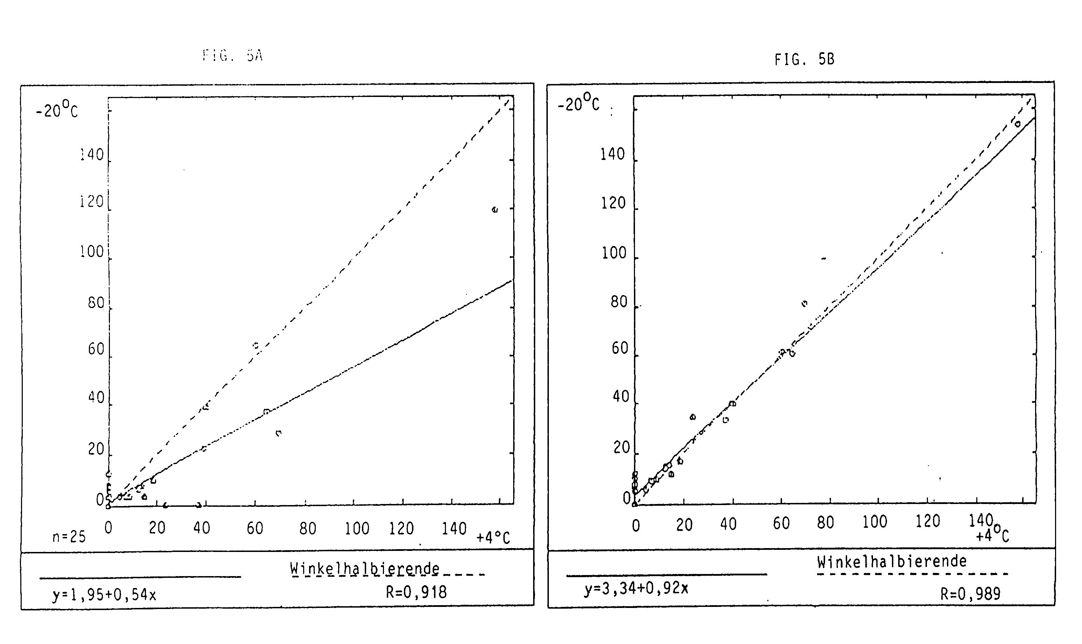

Durch Zugabe von Prolin konnte nicht nur der Korrelationskoeffizient auf R = 0,989 verbessert werden, auch die Geradengleichung y = 3,34 + 0,92x zeigt eine fast vollkommene Übereinstimmung der Methoden an (Fig. 5B); y = 3,52 + 1,09x für die turbidimetrische Bestimmung in Anwesenheit von Prolin; Fig. 4B).

## Patentansprüche

1. Probenaufbereitungsmittel für lipoproteinhältige Proben und zur immunchemischen Bestimmung eines Antigens, dadurch gekennzeichnet, daß das Mittel einen Verdünnungspuffer mit einem Gehalt an einer Aminosäure, gegebenenfalls mit einem Gehalt an einem Reaktionsbeschleuniger, und Antikörper gegen das zu bestimmende Antigen enthält.

2. Probenaufbereitungsmittel für lipoproteinhältige Proben und zur immunchemischen Bestimmung eines Lipoproteins, dadurch gekennzeichnet, daß das Mittel einen Verdünnungspuffer mit einem Gehalt an einer Aminosäure, gegebenenfalls mit einem Gehalt an einem Reaktionsbeschleuniger, und Antikörper gegen das zu bestimmende Lipoprotein enthält.

3. Probenaufbereitungsmittel für Lp(a)-hältige Proben und zur immunchemischen Bestimmung von Lp(a), dadurch gekennzeichnet, daß das Mittel einen Verdünnungspuffer mit einem Gehalt an einer Aminosäure, gegebenenfalls mit einem Gehalt an einem Reaktionsbeschleuniger, und Antikörper gegen Lp(a) enthält.

4. Probenaufbereitungsmittel nach einem der Ansprüche 1 bis 3, dadurch gekennzeichnet, daß die Aminosäure in einer Konzentration von 0,05 bis 3 M, vorzugsweise in einer Konzentration von 0,1 bis 0,5 M, enthalten ist.

5. Probenaufbereitungsmittel nach einem der Ansprüche 1 bis 4, dadurch gekennzeichnet, daß die Aminosäure Prolin ist.

6. Probenaufbereitungsmittel nach einem der Ansprüche 1 bis 5, dadurch gekennzeichnet, daß das Mittel Polyethylenglycol als Reaktionsbeschleuniger enthält.

7. Set zur Probenaufbereitung für eine lipoproteinhältige Probe und zur immunchemischen Bestimmung eines darin enthaltenen Antigens, dadurch gekennzeichnet, daß eine erste Komponente des Mittels einen Gehalt an Puffersubstanzen, an einer Aminosäure und gegebenenfalls an einem Reaktionsbeschleuniger aufweist, und eine zweite Komponente des Mittels Antikörper gegen das zu bestimmende Antigen enthält.

8. Set nach Anspruch 7, dadurch gekennzeichnet, daß die Aminosäure in einer Konzentration von 0,05 bis 3 M, vorzugsweise von 0,1 bis 0,5 M, in der ersten Komponente des Mittels enthalten ist.

9. Set nach einem der Ansprüche 7 oder 8, dadurch gekennzeichnet, daß die Aminosäure Prolin ist.

10. Set nach einem der Ansprüche 7 bis 9, dadurch gekennzeichnet, daß die erste Komponente des Mittels Polyethylenglycol als Reaktionsbeschleuniger enthält.

11. Set nach einem der Ansprüche 7 bis 10, dadurch gekennzeichnet, daß die zweite Komponente des Mittels Antikörper gegen ein Lipoprotein enthält.

12. Set nach einem der Ansprüche 7 bis 11, dadurch gekennzeichnet, daß die zweite Komponente des Mittels Antikörper gegen Lp(a) enthält.

13. Verfahren zur immunchemischen Bestimmung eines Antigens, dadurch gekennzeichnet, daß einer zur immunchemischen Bestimmung vorgesehenen Probe eine Aminosäure zugesetzt wird, anschließend oder gleichzeitig Antikörper gegen das zu bestimmende Antigen in die Probe eingebracht werden und der gebildete Antikörper-Antigen-Komplex bestimmt wird.

14. Verfahren zur Verringerung oder Beseitigung von Trübungen in einer trüben, biologischen Probe, dadurch gekennzeichnet, daß der trüben, biologischen Probe eine Aminosäure zugesetzt wird.

15. Verfahren nach einem der Ansprüche 13 oder 14, dadurch gekennzeichnet, daß die Aminosäure Prolin ist.

16. Verfahren nach einem der Ansprüche 13 bis 15, dadurch gekennzeichnet, daß die Probe eine Blut-, Serum- oder Plasmaprobe ist, welche gegebenenfalls durch einen Lipoproteingehalt optisch trüb ist.

17. Verwendung einer Aminosäure zur Aufbereitung von lipoproteinhältigen Proben, vorzugsweise einer zur immunchemischen Bestimmung eines Antigens vorgesehenen Probe.

18. Verwendung von Prolin zur Aufbereitung von lipoproteinhältigen Proben, vorzugsweise einer zur immunchemischen Bestimmung eines Antigens vorgesehenen Probe.

19. Verwendung einer Aminosäure zur Verringerung oder Beseitigung von Trübungen in einer biologischen Flüssigkeit, vorzugsweise in einer zur immunchemischen Bestimmung vorgesehenen trüben, lipoproteinhältigen Probe eines Antigens.

20. Verwendung von Prolin zur Verringerung oder Beseitigung von Trübungen in einer biologischen Flüssigkeit, vorzugsweise in einer zur immunchemischen Bestimmung vorgesehenen trüben, lipoproteinhältigen Probe eines Antigens.

## Claims

1. Sample treatment means for lipoprotein-containing samples and for the immunochemical determination of an antigen, characterised in that the means contain a dilution buffer with a content of an amino acid, optionally with a content of a reaction accelerator, and antibodies against the antigens to be determined.

2. Sample treatment means for lipoprotein-containing samples and for immunochemical determination of a lipoprotein, characterised in that the means contain a dilution buffer with a content of an amino acid, optionally with a content of a reaction accelerator, and antibodies against the lipoprotein to be detected.

3. Sample treatment means for Lp(a)-containing samples and for immunochemical determination of Lp(a), characterised in that the means contain a dilution buffer with a content of an amino acid, optionally with a content of reaction accelerator, and antibodies against Lp(a).

4. Sample treatment means according to one of Claims 1 to 3, characterised in that the amino acid is contained at a concentration of 0.05 to 3 M, preferably in a concentration of 0.1 to 0.5 M.

5. Sample treatment means according to one of Claims 1 to 4, characterised in that the amino acid is proline.

6. Sample treatment means according to one of Claims 1 to 5, characterised in that the means contains polyethylene glycol as reaction accelerator.

7. Set for sample treatment for a lipoprotein-containing sample and for immunochemical determination of an antigen contained therein, characterised in that a first component of the means has a content of buffer substance, of an amino acid and optionally of a reaction accelerator and a second component of the means contains antibodies against the antigen to be detected.

8. Set according to Claim 7, characterised in that the amino acid is contained in a concentration of 0.05 to 3 M, preferably from 0.1 to 0.5 M in the first component of the means.

9. Set according to one of Claims 7 or 8, characterised in that the amino acid is proline.

10. Set according to one of Claims 7 to 9, characterised in that the first component of the means contains polyethylene glycol as reaction accelerator.

11. Set according to one of Claims 7 to 10, characterised in that the second component of the means contains antibodies against a lipoprotein.

12. Set according to one of Claims 7 to 11, characterised in that the second component of the means contains antibodies against Lp(a).

13. Process for immunochemical determination of an antigen, characterised in that an amino acid is added to a sample provided for the immunochemical determination, then or simultaneously antibodies against the antigen to be determined are introduced into the sample and the antibody antigen complex formed is determined.

14. Process for diminishing or avoiding clouding in a cloudy biological sample, characterised in that an amino acid is added to the cloudy biological sample.

15. Process according to one of Claims 13 or 14, characterised in that the amino acid is proline.

16. Process according to one of Claims 13 to 15, characterised in that the sample is a blood, serum or plasma sample which, in some circumstances, is optically cloudy because of a lipoprotein content.

17. Use of an amino acid for treating lipoprotein-containing samples, preferably a sample provided for the immunochemical determination of an antigen.

18. Use of proline for treating lipoprotein-containing samples, preferably a sample provided for the immunochemical determination of an antigen.

19. Use of an amino acid for reducing or avoiding cloudiness in a biological liquid, preferably in a cloudy lipoprotein-containing sample provided for the immunochemical determination of an antigen.

20. Use of proline for reducing or avoiding cloudiness in a biological liquid, preferably in a cloudy lipoprotein-containing sample provided for the immunochemical determination of an antigen.

## Revendications

1. Agent de traitement d'échantillons destiné à des échantillons contenant des lipoprotéines et à l'analyse immunochimique d'un antigène, caractérisé en ce que l'agent contient un tampon de dilution présentant une teneur en un acide aminé, le cas échéant avec une teneur en un accélérateur de la réaction, et des anticorps contre l'antigène à analyser.

2. Agent de traitement d'échantillons destiné à des échantillons contenant des lipoprotéines et à l'analyse immunochimique d'une lipoprotéine, caractérisé en ce que l'agent contient un tampon de dilution présentant une teneur en un acide aminé, le cas échéant avec une teneur en un accélérateur de la réaction, et des anticorps contre la lipoprotéine à analyser.

3. Agent de traitement d'échantillons destiné à des échantillons contenant des Lp(a) et à l'analyse immunochimique de Lp(a), caractérisé en ce que l'agent contient un tampon de dilution présentant une teneur en un acide aminé, le cas échéant avec une teneur en un accélérateur de la réaction, et des anticorps contre Lp(a).

4. Agent de traitement d'échantillons selon l'une des revendications 1 à 3, caractérisé en ce que l'acide aminé est contenu dans une concentration comprise entre 0,05 et 3 M, de préférence dans une concentration comprise entre 0,1 et 0,5 M.

5. Agent de traitement d'échantillons selon l'une des revendications 1 à 4, caractérisé en ce que l'acide aminé est la proline.

6. Agent de traitement d'échantillons selon l'une des revendications 1 à 5, caractérisé en ce que l'agent contient du polyéthylèneglycol en tant qu'accélérateur de la réaction.

7. Kit de traitement d'échantillons destiné à un échantillon contenant des lipoprotéines et à l'analyse immunochimique d'un antigène contenu dedans, caractérisé en ce qu'une première composante de l'agent présente une teneur en substances tampon, en un acide aminé et le cas échéant en un accélérateur de la réaction, et qu'une seconde composante de l'agent contient des anticorps contre l'antigène à analyser.

8. Kit selon la revendication 7, caractérisé en ce que l'acide aminé est contenu dans la première composante de l'agent dans une concentration comprise entre 0,05 et 3 M, de préférence entre 0,1 et 0,5 M.

9. Kit selon l'une des revendications 7 ou 8, caractérisé en ce que l'acide aminé est la proline.

10. Kit selon l'une des revendications 7 à 9, caractérisé en ce que la première composante de l'agent contient du polyéthylèneglycol en tant qu'accélérateur de la réaction.

11. Kit selon l'une des revendications 7 à 10, caractérisé en ce que la seconde composante de l'agent contient des anticorps contre une lipoprotéine.

12. Kit selon l'une des revendications 7 à 11, caractérisé en ce que la seconde composante de l'agent contient des anticorps contre Lp(a).

13. Procédé d'analyse immunochimique d'un antigène, caractérisé en ce que l'on ajoute un acide aminé à un échantillon destiné à une analyse immunochimique, en ce que l'on applique ensuite ou en même temps dans l'échantillon des anticorps contre l'antigène à analyser et que l'on analyse le complexe anticorps-antigène formé.

14. Procédé de réduction ou d'élimination de troubles dans un échantillon biologique trouble, caractérisé en ce que l'on ajoute un acide aminé à l'échantillon biologique trouble.

15. Procédé selon l'une des revendications 13 ou 14, caractérisé en ce que l'acide aminé est la proline.

16. Procédé selon l'une des revendications 13 à 15, caractérisé en ce que l'échantillon est un échantillon de sang, de sérum ou de plasma éventuellement trouble sur le plan optique par une teneur en lipoprotéines.

17. Utilisation d'un acide aminé pour traiter des échantillons contenant des lipoprotéines, de préférence un échantillon destiné à l'analyse immunochimique d'un antigène.

18. Utilisation de proline pour traiter des échantillons contenant des lipoprotéines, de préférence un échantillon destiné à l'analyse immunochimique d'un antigène.

19. Utilisation d'un acide aminé pour réduire ou éliminer des troubles dans un fluide biologique trouble, de préférence dans un échantillon trouble, contenant des lipoprotéines et destiné à une analyse immunochimique, d'un antigène.

20. Utilisation de proline pour réduire ou éliminer les troubles dans un fluide biologique trouble, de préférence dans un échantillon trouble, contenant des lipoprotéines et destiné à une analyse immunochimique, d'un antigène.
